# EUROPEAN PATENT APPLICATION

(11) **EP 4 036 564 A1**
(43) Date of publication of application: **03.08.2022**
(21) Application number: 20868962.0
(22) Date of filing: 17.09.2020
(51) Int. Cl.: G01N 27/12

(54) **METHOD FOR PREPARING AND METHOD FOR USING GRAPHENE MATERIAL-BASED RESISTIVE GAS SENSOR ARRAY**

(30) Priority: 29.09.2019 CN 201910930862
(71) Applicant: Hangzhou Well-Healthcare Technologies Co., Ltd., Binjiang District Hangzhou, Zhejiang 310000 (CN)
(72) Inventor: WU, Jianmin, Hangzhou, Zhejiang 310000 (CN); CHEN, Qiaofen, Hangzhou, Zhejiang 310000 (CN)
(74) Representative: De Clercq & Partners
(86) International application number: PCT/CN2020/115899
(87) International publication number: WO 2021/057590

(57) **Abstract**

The disclosure provides a preparation method for a graphene material-based resistive gas sensor array and an application method thereof. The preparation method includes: adding a metal salt solution to a graphene oxide solution to obtain a mixed suspension, adjusting a pH of the mixed suspension and dispersing the mixed suspension under ultrasound, incubating the mixed suspension on a shaker, then washing it with deionized water followed by dispersing it in a deionized water to obtain metal ion-induced graphene oxide self-assembled suspension, and preparing a plurality of parts of the suspension by varying the preparation conditions; and adding the plurality of parts of metal ion-induced graphene oxide self-assembled suspension respectively to fingers of a multi-site interdigitated electrode array, and drying naturally, reducing the plurality of parts of the suspension at 60 to 120°C for 3 to 30min. The disclosure achieves uniform loading of a graphene material on a substrate.

## Description

### Technical Field

The present disclosure relates to a preparation method for a graphene material-based resistive gas sensor array and an application method thereof.

### Background

Exhaled breath elucidates the physiological basis of exchange of gas between air and blood, which may reflect the holistic state of an individual. Compared with traditional biochemical tests, such as blood, urine, biopsy and imaging, exhaled breath analysis (EB analysis) is a completely noninvasive diagnostic method with unlimited sample sources and may be used in fields such as disease screening and personal healthcare management. As early as 1999, Phillips et al. observed more than 3,000 volatile organic compounds in human exhaled breath, and the list is growing continuously. Acetone and isoprene are two most ubiquitous endogenous volatile organic compounds according to numerous researches. Herein the acetone is related to glucose metabolism and lipolysis, and the concentration of the isoprene is related to cholesterol biosynthesis. In addition, there are also some potential biomarkers of the inorganic compounds in the exhaled breath that are related to the human metabolism. For example, the concentrations of ammonia and hydrogen sulfide are related to the metabolism of nitrogen/sulfur compounds in vivo. However, for most diseases, there are no specific biomarkers in exhaled breath. We can only observe disease-related difference in overall fingerprints, including the type, concentration and relative abundance of specific gas molecules. From this point of view, pattern recognition may be applied to analyze the exhaled breath to distinguish diseases. Lung cancer, a worldwide leading cause of cancer mortality, is an example. In 2012, Hossam Haick's group reviewed the composition of organic volatiles in exhaled breath of lung cancer patients and the biochemical pathways, pointing out that four types of endogenous organic volatiles in exhaled breath differ most between healthy individuals and patients, including hydrocarbons (alkanes/branched alkanes/branched olefins), primary/secondary alcohols, aldehydes/branched aldehydes, and ketones.

The concentration of the endogenous organic and inorganic volatile compounds in the exhaled breath is at the level of ppm (10⁻⁶), ppb (10⁻⁹) or even lower. At present, technologies for EB analysis can be classified into two categories. One is based on large-scale devices, such as gas chromatography-mass spectrometry (GC-MS), proton transfer reaction mass spectrometry (PTR-MS), selected ion flow tube mass spectrometry (SIFT-MS). The other is based on miniaturized devices, such as micro gas chromatography, colorimetric sensor, electronic nose system. Among them, electronic nose system is the most promising in vitro diagnostic technology to develop a portable and cost-effective noninvasive diagnostic platform for the diagnosis of respiratory and systemic diseases. The core element of the electronic nose system is gas sensors. In numerous electrical gas sensors, resistive gas sensors are most expected to be applied in practical detection. However, most of the current highly sensitive resistive gas sensors are based on semiconductor metal oxides and operated at high temperature. This hinders their applications in portable consumer electronics. Nanomaterials are ideal materials for gas detection especially in low concentration, owing to the significant surface effect and the optoelectronic properties thereof which can be highly sensitive to surface adsorbed substances. Graphene is an attractive nanomaterial for constructing gas sensor with strong electron transmission capability, adjustable band gap, large specific surface area and high flexibility. Besides sensitivity, selectivity is another essential feature in the identification of complex gas. A single sensing element often fails to meet this need. An effective strategy is to use a variety of highly sensitive gas-sensing materials, and design an array to achieve cross-reactive responses, followed by pattern recognition to distinguish complex gas.

### Summary

The present disclosure provides a preparation method for a graphene material-based resistive gas sensor array and an application method thereof, and adopts the following technical schemes.

A preparation method for a graphene material-based resistive gas sensor array, includes the following steps:
Step 1, preparation of a plurality of parts of self-assembled graphene oxide suspension: add a metal salt solution to a graphene oxide solution to obtain a mixed suspension, adjust a pH value of the mixed suspension and then perform an ultrasonic dispersion, incubate the mixed suspension on a shaker for 4 to 12h, wash it with a deionized water until neutral, and then redisperse it in a deionized water to obtain a metal ion-induced graphene oxide self-assembled suspension, change a ratio of the metal salt solution to the graphene oxide solution or change the type of the metal salt solution and repeat the above process to prepare a plurality of parts of the graphene oxide self-assembled suspension, where the number of parts of the graphene oxide self-assembled suspension is the same as the number of sites of a multi-site interdigitated electrode array.
Step 2, preparation of a graphene material-based resistive gas sensor array: add the graphene oxide self-assembled suspension prepared in the step 1 to fingers of the multi-site interdigitated electrode array and dry naturally, place the multi-site interdigitated electrode array in a reductive atmosphere at 60 to 120°C for 3 to 30min to obtain the graphene material-based resistive gas sensor array.

More specifically, the metal salt solution is chloride salt solution of high-valent metal ions.

Further, the metal salt solution is cobalt chloride solution. A specific method of the step 1 is as follows: add the cobalt chloride solution to the graphene oxide solution to obtain the mixed suspension, where a ratio of the graphene oxide to cobalt chloride in the mixed suspension is 1mg/mL: (25 to 75mM), adjust the pH value of the mixed suspension to 7.0 to 9.0 and then perform the ultrasonic dispersion, incubate the mixed suspension on the shaker for 4 to 12h and then wash it with the deionized water until neutral, and redisperse it in the deionized water to obtain the graphene oxide self-assembled suspension, change the ratio of the cobalt chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

Further, the metal salt solution is ferric chloride solution. A specific method of the step 1 is as follows: add the ferric chloride solution to the graphene oxide solution to obtain the mixed suspension, where the ratio of the graphene oxide to ferric chloride in the mixed suspension is 1mg/mL: (5 to 50mM), adjust the pH value of the mixed suspension to 3.0 to 3.5 and then perform the ultrasonic dispersion, incubate the mixed suspension on the shaker for 4 to 12h and then wash it with the deionized water until neutral, and redisperse it in deionized water to obtain the graphene oxide self-assembled suspension, change the ratio of the ferric chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

Further, the metal salt solution is copper chloride solution. A specific method of the step 1 is as follows: add the copper chloride solution to graphene oxide solution to obtain the mixed suspension, where the ratio of the graphene oxide to copper chloride in the mixed suspension is 1mg/mL: (25 to 75mM), adjust the pH value of the mixed suspension to 7.0 to 8.0 and then perform the ultrasonic dispersion, incubate the mixed suspension on the shaker for 4 to 12h and then wash it with deionized water until neutral, and redisperse it in deionized water to obtain the graphene oxide self-assembled suspension, change the ratio of the copper chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

Further, the metal salt solution is cerium chloride solution. A specific method of the step 1 is as follows: add the cerium chloride solution to graphene oxide solution to obtain the mixed suspension, where the ratio of the graphene oxide to cerium chloride in the mixed suspension is 1mg/mL: (10 to 50mM), adjust the pH value of the mixed suspension to 8.0 to 9.0 and then perform the ultrasonic dispersion, incubate the mixed suspension on the shaker for 4 to 12h and then wash it with deionized water until neutral, and redisperse it in the deionized water to obtain the graphene oxide self-assembled suspension, change the ratio of the cerium chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

Further, a hydrochloric acid solution and/or a sodium hydroxide solution is used to adjust the pH value of the mixed suspension.

The reductive atmosphere is a hydrazine hydrate vapor.

An application method of a graphene material-based resistive gas sensor array, includes the following steps.

Put the graphene material-based resistive gas sensor array prepared by the above preparation method for the graphene material-based resistive gas sensor array in a gas flow cell, connect the graphene material-based resistive gas sensor array, read and record a resistance value flowing through each site of the graphene material-based resistive gas sensor array by a multi-channel resistance analyzer.

Firstly feed a carrier gas to the gas flow cell until the resistance value tested by the multi-channel resistance analyzer tends to be stable;
then feed a gas analyte at the same flow rate; and
test the resistance value flowing through each site of the graphene material-based resistive gas sensor array by the multi-channel resistance analyzer to obtain data of a relationship between the resistance value and time, acquire a response value of each site of the graphene material-based resistive gas sensor array to the gas analyte, and perform a statistical analysis according to the response values of the different sites to identify the gas analyte.

Further, the analyte gas is a single-component gas.

The single-component gas is organic volatile gas or inorganic volatile gas.

Further, the gas analyte is a mixed gas, and it includes: a complex environment gas, a human exhaled breath, and a volatile gas produced by metabolism of microorganisms or cells.

In conclusion, the present disclosure provides a preparation method for a graphene material-based resistive gas sensor array. A plurality of metal ion-induced graphene oxide self-assembled materials is integrated in a gas sensor array, to prepare the resistive gas sensor array. Compared with the prior art, the present disclosure has the following positive effects:
1. The present disclosure adopts the high-valent metal ions to induce the graphene oxide's self-assembly, a cross-linked network is formed between the metal ions and the graphene oxide, and through a strategy that the material loading is completed prior to the solvent volatilization, the uniform loading of the graphene material on the substrate is achieved, the coffee ring effect produced by an ordinary drop coating method in preparation of a graphene film is overcome, and at the same time, the detection of trace amount volatile compounds at a room temperature is achieved.
2. The present disclosure adopts the high-valent metal ions to induce the self-assembly of the graphene oxide, and in this process, the surface modification of the graphene oxide is achieved, and at the same time, the graphene loaded on the substrate also has a higher specific surface area, it is beneficial to the adsorption and diffusion of the gas analyte, and the detection of trace organic and inorganic volatile compounds at the room temperature is achieved.
3. The present disclosure integrates different metal-ion-induced self-assembled graphene oxide composites into a same gas sensor array, which can produce cross-reactive responses and thus improves the identification ability towards the gas analytes. The array is easy to prepare, low in cost, and excellent in gas sensing performance that can be applied for the gas identification and differentiation. Further, it may be used in a point-of-care testing (POCT) device for exhaled breath analysis and for disease screening and personalized healthcare management, thus accelerate the process of 4P medicine (preventive, predictive, personalized, and participatory). In addition, it may be used for the monitoring for food volatile metabolic compounds and complex environmental gas to ensure food safety and environmental safety.

### Brief Description of the Drawings

Fig. 1 is a schematic diagram of a graphene material-based gas sensor array of the present disclosure.
Fig. 2 is a scanning electron microscope diagram of a graphene composites in Embodiment 1 to Embodiment 4.
Fig. 3 is a dynamic response diagram of the graphene material-based gas sensor array to different concentrations of an acetone standard gas.
Fig. 4 shows response-concentration curves of the graphene material-based gas sensor array to organic and inorganic volatile gas.
Fig. 5 shows principal component analysis results of the graphene material-based gas sensor array to acetone, isoprene, ammonia and hydrogen sulfide.
Fig. 6 is a box diagram of response of the sites in the graphene material-based gas sensor array to exhaled breath of healthy individuals, patients with lung tumor, and patients after a lung tumor surgery.
Fig. 7 shows discriminant analysis result of the graphene material-based gas sensor array to exhaled breath of the healthy individuals, patients with lung tumor, and patients after lung tumor surgery.

### Detailed Description of the Embodiments

The present disclosure is described in detail below with reference to drawings and specific embodiments.

The present disclosure discloses a preparation method for a graphene material-based resistive gas sensor array, specifically including the following steps:
Step 1, add a metal salt solution to a graphene oxide solution to obtain a mixed suspension, adjust a pH value of the mixed suspension and then perform ultrasonic dispersion, incubate the mixed suspension on a shaker for 4 to 12h, wash it with the deionized water until neutral, and then disperse it with the deionized water to obtain metal ion-induced graphene oxide self-assembled suspension, change the ratio of the metal salt solution to the graphene oxide solution or change the type of the metal salt solution and repeat the above process to prepare a plurality of parts of the graphene oxide self-assembled suspension, where the number of parts of the graphene oxide self-assembled suspension is the same as the number of sites of a multi-site interdigitated electrode array. The plurality of parts of the graphene oxide self-assembled suspension is obtained by adding the different metal salt solution or change the ratio of the metal salt solution to the graphene oxide solution. Therefore, the plurality of parts of the graphene oxide self-assembled suspension obtained finally is different from each other.
Step 2, add the graphene oxide self-assembled suspension prepared in the step 1 to fingers of the multi-site interdigitated electrode array and dry naturally, place the multi-site interdigitated electrode array in a reductive atmosphere and reduce at 60 to 120°C for 3 to 30 min to obtain the graphene material-based resistive gas sensor array. Because the plurality of parts of the graphene oxide self-assembled suspension prepared in the step 1 is different from each other, the compositions of the graphene material at each site of the prepared graphene material-based resistive gas sensor array are different from each other.

Specifically, in the step 1, the metal salt solution is chloride salt solution of high-valent metal ions, includes, but is not limited to a ferric chloride, a cobalt chloride, a nickel chloride, a copper chloride, a tin chloride, a cerium chloride and the like, and the pH value of the mixed suspension is adjusted by hydrochloric acid solution and/or sodium hydroxide solution until it meets the requirements.

While the metal salt solution is cobalt chloride solution, the step 1 is specifically as follows: add the cobalt chloride solution to the graphene oxide solution to obtain a mixed suspension, where the ratio of the graphene oxide to cobalt chloride in the mixed suspension is 1mg/mL: (25 to 75mM), adjust the pH value of the mixed suspension to 7.0 to 9.0 and then perform the ultrasonic dispersion, incubate the mixed suspension on the shaker for 4 to 12h and then wash it with the deionized water until neutral, and disperse it in the deionized water to obtain the graphene oxide self-assembled suspension, change the ratio of the cobalt chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

While the metal salt solution is ferric chloride solution and obtain a mixed suspension, the step 1 is specifically as follows: add the ferric chloride solution to the graphene oxide solution, where the ratio of the graphene oxide to ferric chloride in the mixed suspension is 1mg/mL: (5 to 50mM), adjust the pH value of the mixed suspension to 3.0 to 3.5 and then perform the ultrasonic dispersion, incubate the mixed suspension on the shaker for 4 to 12 h and then wash it with the deionized water until neutral, and disperse it in the deionized water to obtain the graphene oxide self-assembled suspension, change the ratio of the ferric chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

While the metal salt solution is copper chloride solution, the step 1 is specifically as follows: add the copper chloride solution to the graphene oxide solution to obtain a mixed suspension, where the ratio of the graphene oxide to copper chloride in the mixed suspension is 1mg/mL: (25 to 75mM), adjust the pH value of the mixed suspension to 7.0 to 8.0 and then perform the ultrasonic dispersion, incubate the mixed suspension on the shaker for 4 to 12 h and then wash it with the deionized water until neutral, and disperse it with the deionized water to obtain the graphene oxide self-assembled suspension, change the ratio of the copper chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

While the metal salt solution is cerium chloride solution, the step 1 is specifically as follows: add the cerium chloride solution to the graphene oxide solution to obtain a mixed suspension, where the ratio of the graphene oxide to cerium chloride in the mixed suspension is 1mg/mL: (10 to 50mM), adjust the pH value of the mixed suspension to 8.0 to 9.0 and then perform the ultrasonic dispersion, incubate the mixed suspension on the shaker for 4 to 12h and then wash it with the deionized water until neutral, and disperse it with the deionized water to obtain the graphene oxide self-assembled suspension, change the ratio of the cerium chloride solution to the graphene oxide solution and repeat the above process to prepare the plurality of parts of the graphene oxide self-assembled suspension, where the number of parts of the graphene oxide self-assembled suspension is the same as the number of sites of the multi-site interdigitated electrode array.

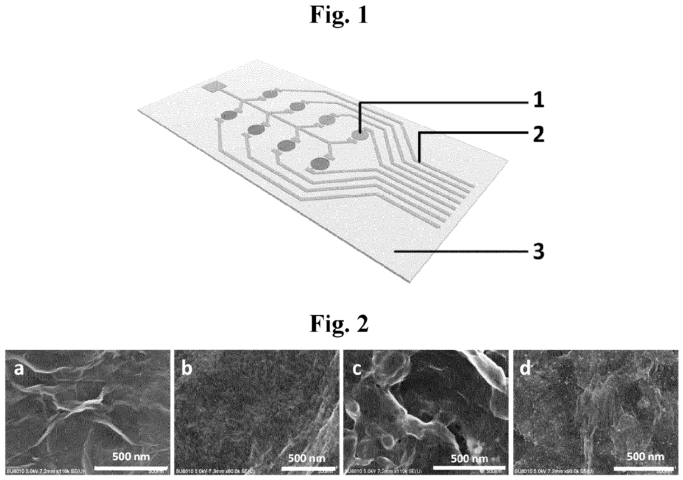

In the step 2, the reductive atmosphere is a hydrazine hydrate vapor. It may be understood that the reductive atmosphere may also be other substances with the similar effects. In addition, the number of the sites of the multi-site interdigitated electrode array may be freely selected according to actual needs. As shown in Fig. 1, it is a schematic diagram of a graphene material-based resistive gas sensor array with 8 sites, including a substrate 1, an external circuit 2 and a graphene material site 3, where the substrate 1 may be: a flexible substrate such as a polyethylene terephthalate (PET) film, a polyethylene naphthalate (PEN) film, a polyimide (PI) film, and a polycarbonate (PC) film, and a hard substrate such as silicon, glass, ceramics and a printed circuit board (PCB).

The following are the specific embodiments of the present disclosure.

### EMBODIMENTS

### Embodiment 1

This embodiment provides a preparation method for preparing a three-site graphene material-based resistive gas sensor array by using cobalt chloride solution, and the specific steps thereof are as follows.

Step 1, add the cobalt chloride solution to the graphene oxide solution, where the ratios of graphene oxide to cobalt chloride in the mixed suspension are 1mg/mL:15mM, 1mg/mL:30mM, and 1mg/mL:45mM respectively, adjust a pH value of three types of the mixed suspension to 7.0 and perform ultrasonic dispersion, incubate the three types of the mixed suspension on a shaker for 8h and wash it with a deionized water until neutral, and then disperse it with a deionized water to obtain the three types of cobalt ion-induced graphene oxide self-assembled suspension.

Step 2, respectively add 5µL of the three types of the cobalt ion-induced graphene oxide self-assembled suspension prepared in the step 1 to fingers of a three-site interdigitated electrode array and dry naturally, where a distance between the fingers is 100µm, place the multi-site interdigitated electrode array in a hydrazine hydrate vapor and reduce at 80°C for 10min, to obtain the three-site graphene material-based resistive gas sensor array. Herein, a scanning electron microscope diagram of the graphene material prepared while the ratio of the graphene oxide to cobalt chloride is 1mg/mL:30mM is shown in Fig. 2a.

### Embodiment 2

This embodiment provides a preparation method for preparing a five-site graphene material-based resistive gas sensor array by using ferric chloride solution, and the specific steps thereof are as follows.

Step 1, add the ferric chloride solution to the graphene oxide solution, where the ratios of graphene oxide to ferric chloride in the mixed suspension are 1mg/mL:5mM, 1mg/mL:10mM, 1mg/mL:20mM, 1mg/mL:30mM, and 1mg/mL:40mM respectively, adjust a pH value of five types of the mixed suspension to 3.0 and perform ultrasonic dispersion, incubate the five types of the mixed suspension on a shaker for 6 h and wash it with a deionized water until neutral, and then disperse it with a deionized water to obtain five types of ferric ion-induced graphene oxide self-assembled suspension.

Step 2, respectively add 2.5µL of the five types of the ferric ion-induced graphene oxide self-assembled suspension prepared in the step 1 to fingers of a five-site interdigitated electrode array and dry naturally, where a distance between the fingers is 100µm, place the multi-site interdigitated electrode array in a hydrazine hydrate vapor and reduce at 80°C for 5min, to obtain the graphene material-based resistive gas sensor array. Herein, a scanning electron microscope diagram of the graphene material prepared while the ratio of the graphene oxide to ferric chloride is 1mg/mL:20mM is shown in Fig. 2b.

### Embodiment 3

This embodiment provides a preparation method for preparing a five-site graphene material-based resistive gas sensor array by using copper chloride solution, and the specific steps thereof are as follows.

Step 1, add the copper chloride solution to the graphene oxide solution, where the ratios of graphene oxide to copper chloride in the mixed suspension are 1mg/mL:15mM, 1mg/mL:20mM, 1mg/mL:30mM, 1mg/mL:40mM, and 1mg/mL:60mM respectively, adjust a pH value of five types of the mixed suspension to 7.5 and perform ultrasonic dispersion, incubate the five types of the mixed suspension on a shaker for 6 h and wash it with a deionized water until neutral, and then disperse it with a deionized water to obtain five types of copper ion-induced graphene oxide self-assembled suspension.

Step 2, respectively add 5µL of the five types of the graphene oxide self-assembled suspension prepared in the step 1 to fingers of a five-site interdigitated electrode array and dry naturally, where a distance between the fingers is 100µm, place the multi-site interdigitated electrode array in a hydrazine hydrate vapor and reduce at 80°C for 10min, to obtain the graphene material-based resistive gas sensor array. Herein, a scanning electron microscope diagram of the graphene material prepared while the ratio of the graphene oxide to copper chloride is 1mg/mL:30mM is shown in Fig. 2c.

### Embodiment 4

This embodiment provides a preparation method for preparing a two-site graphene material-based resistive gas sensor array by using cerium chloride solution, and the specific steps thereof are as follows.

Step 1, add 10µL of the cerium chloride solution to the graphene oxide solution, where the ratios of graphene oxide to cerium chloride in the mixed suspension are 1mg/mL:10mM, and 1mg/mL:20mM respectively, adjust a pH value of two types of the mixed suspension to 8.0 and perform ultrasonic dispersion, incubate the two types of the mixed suspension on a shaker for 5h and wash with deionized water until neutral, and then disperse it with the deionized water to obtain two types of cerium ion-induced graphene oxide self-assembled suspension.

Step 2, respectively add the two types of the graphene oxide self-assembled suspension prepared in the step 1 to fingers of a two-site interdigitated electrode array and dry naturally, where a distance between the fingers is 100µm, place the multi-site interdigitated electrode array in a hydrazine hydrate vapor and reduce at 80°C for 15min, to obtain the graphene material-based resistive gas sensor array. Herein, a scanning electron microscope diagram of the graphene material prepared while the ratio of the graphene oxide to cerium chloride is 1mg/mL:20mM is shown in Fig. 2d.

The above embodiments describe the detailed steps for preparing the graphene material-based resistive gas sensor array by the preparation method for preparing the graphene material-based resistive gas sensor array of the present disclosure. The present disclosure further introduces an application method of the graphene material-based resistive gas sensor array, specifically including the following steps:
1. Put the graphene material-based resistive gas sensor array in a gas flow cell, and connect the array to a multi-channel resistance analyzer which can read and record the resistance of each site of the array. Herein, the carrier gas is a background gas of the gas analyte. The gas analyte includes but is not limited to single-component gas or mixed gas. The single-component gas is organic and inorganic volatile gas, includes but not limited to acetone, isoprene, ethanol, ammonia, hydrogen sulfide, water vapor, carbon monoxide, carbon dioxide, nitric dioxide and nitrogen dioxide and the like. The mixed gas may be human exhaled breath, other types of metabolic gas, and complex environmental gas. The response value may be defined as *R%=[(I-I₀)*/*I₀*×*100]*, where I is a current measured by the gas analyte flowing through the sensor under a constant voltage, and *I₀* is a current measured while the carrier gas flows through the sensor.
2. Feed the carrier gas to the gas flow cell until the resistance value detected by the multi-channel resistance analyzer tends to be stable.
3. Feed the gas analyte at the same flow rate.
4. Test the resistance value flowing through each site of the graphene material-based resistive gas sensor array by the multi-channel resistance analyzer to obtain data of a relationship between the resistance value and time, acquire a response value of each site of the graphene material-based resistive gas sensor array to the gas analyte, and perform a statistical analysis according to the response values of the different sites to identify the gas analyte. Herein, the statistical analysis includes but is not limited to principal component analysis, discriminant analysis, artificial neural network and the like.

Specifically, in one implementation, that the eight-site graphene material-based resistive gas sensor array prepared in the different ratios is applied for single-component gas analysis: put the array in the gas flow cell and connect the array to the multi-channel resistance analyzer, feed carrier gas to the gas flow cell until the multi-channel resistance analyzer tends to be stable, and then feed the different concentrations of the acetone, or isoprene, or ammonia, or hydrogen sulfide at the same flow rate. Under the voltage of 0.1V, the resistance value flowing through each site of the array is tested to obtain the data of the relationship between the current value and the time, thus the response of each site of the array to the different gas analytes is known. In this embodiment, the composites of each site of the eight-site graphene material-based resistive gas sensor array are a graphene material prepared by the graphene oxide and cobalt chloride in the ratio of 1mg/mL:30mM, a graphene material prepared by the graphene oxide and ferric chloride in the ratio of 1mg/mL:10mM, a graphene material prepared by the graphene oxide and ferric chloride in the ratio of 1mg/mL:20mM, a graphene material prepared by the graphene oxide and ferric chloride in the ratio of 1mg/mL:30mM, a graphene material prepared by the graphene oxide and copper chloride in the ratio of 1mg/mL:20mM, a graphene material prepared by the graphene oxide and copper chloride in the ratio of 1mg/mL:30mM, a graphene material prepared by the graphene oxide and copper chloride in the ratio of 1mg/mL:40mM, and a graphene material prepared by the graphene oxide, and cerium chloride in the ratio of 1mg/mL:20mM.

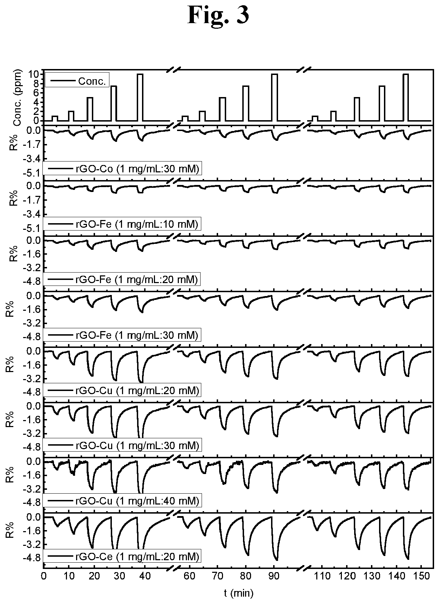

A kinetic curve in acetone standard gas analysis is shown in Fig. 3. The three cyclic tests towards 1, 2, 5, 7.5, and 10ppm acetone standard gas indicate that the prepared eight-site gas sensor arrays with different compositions not only have the high sensitivity, but also have the good response reproducibility.

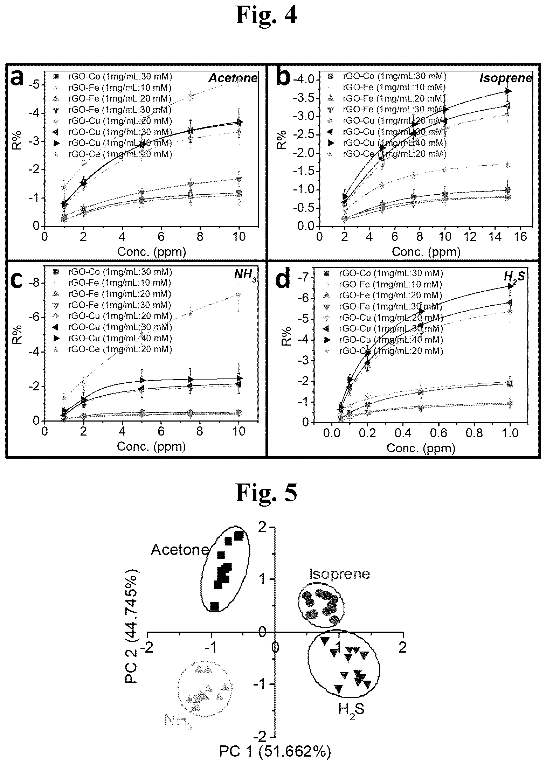

Fig. 4a to Fig. 4d show response-concentration curves of the graphene material-based gas sensor array to organic and inorganic volatile gas. Fig. 4a shows response-concentration curves to 1 to 10ppm acetone standard gas, Fig.4b shows response-concentration curves to 2 to 15ppm isoprene standard gas, Fig.4c shows response-concentration curves to 1 to 10ppm ammonia (NH₃) standard gas, and Fig.4d shows response-concentration curves to 0.05 to 1ppm hydrogen sulfide (H₂S) standard gas. Test results indicate that the eight sites in the array may respond to all four gas, but the sensitivities are different respectively. This interactive response mode may be used to identify and distinguish the different gas. After the response values are normalized, the principal component analysis is performed. Fig. 5 shows that in a two-dimensional principal component analysis (PCA) diagram, the four gas analytes are distributed in four quadrants and are well distinguished.

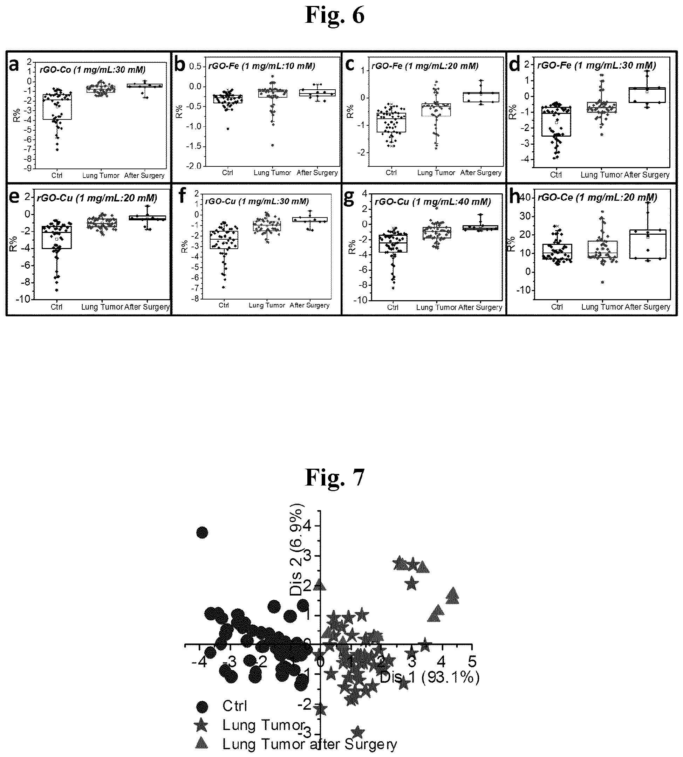

In another implementation, the eight-site graphene material-based resistive gas sensor array is applied for human exhaled breath analysis. Firstly, prepare the gas analyte. Collect exhaled breath from fasting patients with lung tumor or healthy individuals into a 2L fluorinated ethylene propylene (FEP) sampling bag in ventilated environment, and collect local ambient gas as the carrier gas at the same time. During the collection process, the gas firstly passes through a 10 to 50cm Teflon pipe with an inner diameter of 2 to 10mm immersed in an ice-water bath. Secondly, test and analyze the gas analyte. Put the eight-site graphene material-based resistive gas sensor array in the gas flow cell and connect it to the multi-channel resistance analyzer, feed carrier gas to the gas flow cell until the multi-channel resistance analyzer tends to be stable, and then feed the pretreated human exhaled breath at the same flow rate. Under the voltage of 0. IV, the resistance value flowing through each site is tested by the multi-channel resistance analyzer, to obtain the data of the relationship between the current value and the time, thus a response image of each site of the array to the different sample gas is acquired. 106 exhaled breath samples of individuals in the same age group are tested, including 48 healthy samples (marked as ctrl in the figure), 48 exhaled breath samples of patients with lung tumor (marked as lung tumor in the figure), and 10 patients after a lung tumor surgery (marked as lung tumor after surgery in the figure), and the response values of the different sites are extracted. Fig. 6a to Fig. 6h are box diagrams of response value of the graphene material-based resistive gas sensor array to exhaled breath of healthy individuals, patient with lung tumor, and patient after lung tumor surgery. According to the response values, a healthy control group and a patient group distributed in two regions in discriminant analysis. As shown in Fig. 7, it is the discriminant analysis results to the exhaled breath of the healthy individuals, patients with lung tumor, and the patients after the lung tumor surgery, which indicates that the array may be used for the screening of lung tumor diseases.

The above shows and describes the basic principles, main features and advantages of the present disclosure. Those skilled in the art should understand that the above embodiments do not limit the present disclosure in any forms, and all the technical schemes obtained by means of equivalent replacements or equivalent transformations fall within a scope of protection of the present disclosure.

## Claims

1. A preparation method for a graphene material-based resistive gas sensor array, comprising the following steps:
Step 1, adding a metal salt solution to graphene oxide solution to obtain a mixed suspension, adjusting a pH value of the mixed suspension and then performing an ultrasonic dispersion, incubating the mixed suspension on a shaker for 4 to 12h, washing it with a deionized water until neutral, and redispersing it in deionized water to obtain a metal ion-induced graphene oxide self-assembled suspension, changing a ratio of the metal salt solution to the graphene oxide solution or changing the type of the metal salt solution and repeating the above process to prepare a plurality of parts of the graphene oxide self-assembled suspension, where the number of parts of the graphene oxide self-assembled suspension is the same as the number of sites of a multi-site interdigitated electrode array;
Step 2, adding the graphene oxide self-assembled suspension prepared in the step 1 to fingers of the multi-site interdigitated electrode array and drying naturally, placing the multi-site interdigitated electrode array in a reductive atmosphere at 60 to 120°C for 3 to 30min to obtain the graphene material-based resistive gas sensor array.

2. The preparation method for the graphene material-based resistive gas sensor array according to claim 1, wherein,
the metal salt solution is a chloride salt solution with high-valent metal ions.

3. The preparation method for the graphene material-based resistive gas sensor array according to claim 2, wherein,
the metal salt solution is a cobalt chloride solution; and
a specific method of the step 1 is as follows: adding the cobalt chloride solution to the graphene oxide solution to obtain the mixed suspension, where a ratio of the graphene oxide to the cobalt chloride in the mixed suspension is 1mg/mL: (25 to 75mM), adjusting the pH value of the mixed suspension to 7.0 to 9.0 and then performing the ultrasonic dispersion, incubating the mixed suspension on the shaker for 4 to 12h and then washing it with the deionized water until neutral, and redispersing it in the deionized water to obtain the graphene oxide self-assembled suspension, changing the ratio of the cobalt chloride solution to the graphene oxide solution and repeating the above process to prepare the plurality of parts of the graphene oxide self-assembled suspension, wherein the number of parts of the graphene oxide self-assembled suspension is the same as the number of sites of the multi-site interdigitated electrode array.

4. The preparation method for the graphene material-based resistive gas sensor array according to claim 2, wherein,
the metal salt solution is a ferric chloride solution; and
a specific method of the step 1 is as follows: adding the ferric chloride solution to the graphene oxide solution to obtain the mixed suspension, where the ratio of the graphene oxide to the ferric chloride in the mixed suspension is 1mg/mL: (5 to 50mM), adjusting the pH value of the mixed suspension to 3.0 to 3.5 and then performing the ultrasonic dispersion, incubating the mixed suspension on the shaker for 4 to 12h and then washing it with the deionized water until neutral, and redispersing it in deionized water to obtain the graphene oxide self-assembled suspension, changing the ratio of the ferric chloride solution to the graphene oxide solution and repeating the above process to prepare the plurality of parts of the graphene oxide self-assembled suspension, wherein the number of parts of the graphene oxide self-assembled suspension is the same as the number of sites of the multi-site interdigitated electrode array.

5. The preparation method for the graphene material-based resistive gas sensor array according to claim 2, wherein,
the metal salt solution is a copper chloride solution; and
a specific method of the step 1 is as follows: adding the copper chloride solution to the graphene oxide solution to obtain a mixed suspension, where the ratio of the graphene oxide to copper chloride in the mixed suspension is 1mg/mL: (25 to 75mM), adjusting the pH value of the mixed suspension to 7.0 to 8.0 and then performing the ultrasonic dispersion, incubating the mixed suspension on the shaker for 4 to 12h and then washing it with the deionized water until neutral, and redispersing it in the deionized water to obtain the graphene oxide self-assembled suspension, changing the ratio of the copper chloride solution to the graphene oxide solution and repeating the above process to prepare the plurality of parts of the graphene oxide self-assembled suspension, wherein the number of parts of the graphene oxide self-assembled suspension is the same as the number of sites of the multi-site interdigitated electrode array.

6. The preparation method for the graphene material-based resistive gas sensor array according to claim 2, wherein,
the metal salt solution is a cerium chloride solution; and
a specific method of the step 1 is as follows: adding the cerium chloride solution to the graphene oxide solution to obtain the mixed suspension, wherein the ratio of the graphene oxide to cerium chloride in the mixed suspension is 1mg/mL: (10 to 50mM), adjusting the pH value of the mixed suspension to 8.0 to 9.0 and then performing the ultrasonic dispersion, incubating the mixed suspension on the shaker for 4 to 12h and then washing it with the deionized water until neutral, and redispersing it in the deionized water to obtain the graphene oxide self-assembled suspension, changing the ratio of the cerium chloride solution to the graphene oxide solution and repeating the above process to prepare the plurality of parts of the graphene oxide self-assembled suspension, wherein the number of parts of the graphene oxide self-assembled suspension is the same as the number of sites of the multi-site interdigitated electrode array.

7. The preparation method for the graphene material-based resistive gas sensor array according to claim 1, wherein,
a hydrochloric acid solution and/or a sodium hydroxide solution is used to adjust the pH value of the mixed suspension; and
the reductive atmosphere is a hydrazine hydrate vapor.

8. An application method of a graphene material-based resistive gas sensor array, comprising the following steps:
putting the graphene material-based resistive gas sensor array prepared by the preparation method for the graphene material-based resistive gas sensor array according to any one of claims 1-7 in a gas flow cell, connecting the graphene material-based resistive gas sensor array, reading and recording a resistance value flowing through each site of the graphene material-based resistive gas sensor array by a multi-channel resistance analyzer;
firstly feeding a carrier gas to the gas flow cell until the resistance value tested by the multi-channel resistance analyzer tends to be stable;
then feeding a gas analyte at the same flow rate; and
testing the resistance value flowing through each site of the graphene material-based resistive gas sensor array by the multi-channel resistance analyzer to obtain data of a relationship between the resistance value and time, acquiring a response value of each site of the graphene material-based resistive gas sensor array to the gas analyte, and performing a statistical analysis according to the response values of the different sites to identify the gas analyte.

9. The application method of the graphene material-based resistive gas sensor array according to claim 8, wherein,
the gas analyte is a single-component gas; and
the single-component gas is organic volatile gas or inorganic volatile gas.

10. The application method of the graphene material-based resistive gas sensor array according to claim 8, wherein,
the gas analyte is a mixed gas, and it includes: a complex environment gas, a human exhaled breath, and a volatile gas produced by metabolism of microorganisms or cells.
